(19) Europäisches Patentamt / European Patent Office / Office européen des brevets

(11) **EP 1 757 283 B1**

(12) **EUROPEAN PATENT SPECIFICATION**

(45) Date of publication and mention
of the grant of the patent:
**17.10.2012 Bulletin 2012/42**

(51) Int Cl.:
*A61K 31/132* *(2006.01)*    *A61K 31/4412* *(2006.01)*
*A61K 31/506* *(2006.01)*    *A61K 31/53* *(2006.01)*
*A61K 33/24* *(2006.01)*    *A61P 35/00* *(2006.01)*
*A61K 31/282* *(2006.01)*

(21) Application number: **05745983.6**

(22) Date of filing: **02.06.2005**

(86) International application number:
**PCT/JP2005/010182**

(87) International publication number:
**WO 2005/120480 (22.12.2005 Gazette 2005/51)**

(54) **ANTITUMOR EFFECT FORTIFIER, ANTITUMOR AGENT AND METHOD OF THERAPY FOR CANCER**

MITTEL ZUR VERSTÄRKUNG VON ANTITUMORALEN WIRKUNGEN, ANTITUMORALES MITTEL UND THERAPIEVERFAHREN BEI KREBS

AGENT RENFORÇANT UN EFFET ANTITUMORAL, AGENT ANTITUMORAL ET PROCÉDÉ DE THÉRAPIE POUR LE CANCER

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **09.06.2004 JP 2004171520**

(43) Date of publication of application:
**28.02.2007 Bulletin 2007/09**

(73) Proprietor: **TAIHO PHARMACEUTICAL CO., LTD.**
**Tokyo 101-0054 (JP)**

(72) Inventors:
• **KOIZUMI, Katsuhisa**
c/o TAIHO PHARMACEUTICAL. CO., LTD.
Tokushima-shi,
Tokushima 7710194 (JP)
• **UCHIDA, Junji**
c/o TAIHO PHARMACEUTICAL. CO., LTD.
Tokushima-shi,
Tokushima 7710194 (JP)
• **TAKECHI, Teiji**
c/o TAIHO PHARMACEUTICAL. CO., LTD.
Tokushima-shi,
Tokushima 7710194 (JP)
• **NUKATSUKA, Mamoru**
c/o TAIHO PHARMACEUTICAL. CO., LTD.
Tokushima-shi,
Tokushima 7710194 (JP)

(74) Representative: **HOFFMANN EITLE**
**Patent- und Rechtsanwälte**
**Arabellastrasse 4**
**81925 München (DE)**

(56) References cited:
**WO-A-03/015521    US-A- 5 716 988**

• **UYAMA T ET AL: "Prophylactic long-term treatment of bladder tumors with oral chemotherapy (tegafur)" UROLOGY, BELLE MEAD, NJ, US, vol. 23, no. 4, 1 April 1984 (1984-04-01), pages 367-369, XP023270671 ISSN: 0090-4295 [retrieved on 1984-04-01]**
• **CHOLLET P ET AL: "Phase II trial with S-1 in chemotherapy-nave patients with gastric cancer. A trial performed by the EORTC Early Clinical Studies Group (ECSG)" EUROPEAN JOURNAL OF CANCER, PERGAMON PRESS, OXFORD, GB, vol. 39, no. 9, 1 June 2003 (2003-06-01), pages 1264-1270, XP004425218 ISSN: 0959-8049**
• **SASAKI T. ET AL: 'Progress in chemotherapy for colorectal cancer.' CANCER & CHEMOTHERAPY. vol. 27, no. 14, December 2000, pages 2185 - 2192, XP002990937**
• **OMURA K. ETA L: 'Treatment of metastatic liver carcinoma: chemotherapy and immunotherapy.' NIPPON GEKA GAKKAI ZASSHI. vol. 105, no. 10, October 2003, pages 730 - 734, XP002990938**

- **CUNNINHAM D. ET AL: 'New options for outpatient chemotherapy- the role of oral fluoropyrimidines.' CANCER TREATMENT REVIEWS. vol. 27, no. 4, 2001, pages 211 - 220, XP002990939**
- **MALET-MARTINO M ET AL: "Clinical studies of three oral prodrugs of 5-fluorouracil (capecitabine, UFT, S-1): a review.", THE ONCOLOGIST 2002 LNKD- PUBMED:12185293, vol. 7, no. 4, 2002, pages 288-323, ISSN: 1083-7159**
- **SCHEITHAUER W ET AL: "The role of oxaliplatin in the management of upper gastrointestinal tract malignancies", COLORECTAL DISEASE 200311 GB LNKD- DOI:10.1046/J.1463-1318.5.S3.5.X, vol. 5, no. SUPPL. 3, November 2003 (2003-11), pages 36-44, ISSN: 1462-8910**

Remarks:

The file contains technical information submitted after the application was filed and not included in this specification

**Description**

TECHNICAL FIELD

The present invention relates to an antitumor effect potentiator and the use thereof in a method for treating cancer by potentiating an antitumor effect according to a novel concomitant administration of an antitumor preparation Further, the present invention relates to a pharmaceutical composition comprising, among others, the present antitumor effect potentiator.

BACKGROUND ART

The research and development of antitumor preparations has been actively carried out. A variety of effective antitumor preparations are clinically used in the treatment of malignant tumors. For example, tegafur is a drug that is activated *in vivo* and gradually releases the active principle, i.e., 5-fluorouracil (hereinafter referred to as "5-FU"), thereby lessening the toxicity and side effects presented by 5-FU.

A compound pharmaceutical agent of this tegafur and uracil (trade name: UFT, molar ratio of tegafur/uracil = 1 : 4, manufactured by Taiho Pharmaceutical Co., Ltd.) is known. This compound pharmaceutical agent exhibits a significant antitumor effect due to the fact that uracil, which does not have any antitumor effect by itself, inhibits the inactivation of 5-FU which when used alone is promptly metabolized and inactivated in vivo.

A 3-membered compound pharmaceutical agent containing tegafur, gimeracil, and oteracil potassium (trade name: TS-1, molar ratio of tegafur/gimeracil/oteracil potassium = 1 : 0.4 : 1, manufactured by Taiho Pharmaceutical Co., Ltd.) is also known. This compound pharmaceutical agent has a stronger antitumor effect due to the fact that gimeracil exhibits a 5-FU decomposition inhibitory action about 200 times greater than that of uracil. With respect to this compound pharmaceutical agent, oteracil potassium specifically inhibits the increase in gastrointestinal toxicity that is likely to be accompanied by the potentiation in antitumor effect attained by the two ingredients, i.e., tegafur and gimeracil, thereby potentiating the therapeutic effect. UFT and TS-1 therefore contribute to the treatment of various malignant tumors.

Drugs and therapeutic methods that give a stronger therapeutic effect are still required such that the survival of cancer patients is further prolonged. One example of a therapeutic method that has long been used to achieve such an object is administering in combination a plurality of drugs that have different mechanisms of expressing antitumor effects and different side effects so as to improve the therapeutic result (a combination therapy). Some combination therapies contribute to improving therapeutic results (see e.g. Japanese Patent Publications No. 2557303 and No. 2614164, and Japanese Unexamined Patent Publications No. 1996-169825 and No. 2002-205945). For example, oxaliplatin when used alone exhibits a low antitumor effect, and is therefore used in combination therapies with other pharmaceutical agents. Combination therapies using 5-fluorouracil and calcium folinate (FOLFOX) are commonly used worldwide (see e.g. Journal of Clinical Oncology, Vol.22, 22-30, 2004; ibid. Viol.21, 2059-2069, 2004, and ibid. Viol.18, 2938-2947, 2000). FOLFOX requires complicated procedures and are thus problematic in being detrimental to the QOL of patients due to the physical restraint accompanied by continuous infusion, high medical costs, etc. Therefore, development of better combination therapies using oxaliplatin has been attempted all over the world. As an example, a combination therapy using oxaliplatin and capecitabine (XELOX), which is a fluorinated pyrimidine-based anticancer agent (trade name: Xeroda), is reported to give an antitumor effect almost identical to FOLFOX (see e.g. Journal of Clinical Oncology, Vol.22, 2084-2091, 2004). However, combination therapies that give a stronger therapeutic effect are still required.

M. Malet-Martino et al., The Oncologist, 7, 288-323 (2002) is a review article on clinical studies of three oral prodrugs of 5-FU. Among these prodrugs, S-1 is a combination of 5-chloro-2,4-dihydroxypyridine (gimeracil), potassium oxonate (oteracil potassium) and 5-Fluoro-1-(tetrahydro-2-furyl)uracil (tegafur). It is reported that in a comparative study of S-1 with or without low-dose cis-diaminedichloroplatinum(II) (cisplatin) for patient with recurrent or advanced gastric cancer showed a significantly superior response rate (RR) but a reduced median survival time (MST) in the S-1/cisplatin group.

W. Scheithauer et al., Colorectal Disease, 5(3), 36-44 (2003) reports that a combination of 5-FU with oxaliplatin shows a synergism in the treatment of colorectal cancer, and that oxaliplatin has a more tolerable side-effect profile than cisplatin, suggesting a better efficacy..

DISCLOSURE OF THE INVENTION

A primary object of the invention is to provide an antitumor effect potentiator for a tegafur/gimeracil/oteracil potassium compound pharmaceutical agent; a use in a method for treating cancer that gives an excellent therapeutic effect due to the use of a specific pharmaceutical agent in combination with the compound pharmaceutical agent; an antitumor preparation containing the specific pharmaceutical agent and the compound pharmaceutical agent; and a kit.

In view of the current state of the art described above, the inventors studied a novel combination therapy with a 3-membered compound pharmaceutical agent containing tegafur, gimeracil, and oteracil potassium in combination with another antitumor preparation to develop a method for treating cancer that strongly contributes to prolonging the survival of patients, and as a result, found that the use of a platinous complex, i.e., cis-oxalate (1R,2R-diaminocyclohexane) platinum(II) (generic name: oxaliplatin, trade names: Eloxatin and Elplat, hereinafter referred to as l-OHP) in combination with the aforementioned 3-membered compound pharmaceutical agent significantly potentiates the antitumor effect without aggravating side effects. The inventors confirmed that the tumor growth inhibitory effect attained by this novel combination therapy is superior to that of standard chemotherapies for cancer of the large intestine such as a combination therapy using a tegafur/uracil compound pharmaceutical agent and a salt of *d,l*-folinic acid (see Japanese Patent Publication No. 2557303) and a combination therapy using a tegafur/uracil compound pharmaceutical agent, a salt of d,l-folinic acid, and 1-OHP (see US 6,602,870). The present invention has been accomplished based on these novel findings.

Thus, the present invention provides cis-oxalate(1R,2R-diaminocyclohexane) platinum(II) for use in a method of potentiating the antitumor activity of a composition comprising

- a therapeutically effective amount of tegafur,

- an antitumor effect potentiating amount of gimeracil and

- a side effect inhibiting effective amount of oteracil potassium.

Also, the present invention provides Cis-oxalate(1R,2R-diaminocyclohexane) platinum(II) in combination with

- a therapeutically effective amount of tegafur,

- an antitumor effect potentiating amount of gimeracil and

- a side effect inhibiting effective amount of oteracil potassium

for use in a method of treating cancer.

Even further, the present invention provides a pharmaceutical composition comprising tegafur, gimeracil, oteracil potassium, and cis-oxalate(*1R,2R*-diaminocyclohexane)platinum(II) as active ingredients in one or more pharmaceutical agent(s) each containing one or more of these active ingredients.

Preferred embodiments of the present invention are as defined in the appended dependent claims and in the detailed description below.

According to the present invention cis-oxalate(1R,2R-diaminocyclohexane)platinum(II) (oxaliplatin, 1-OHP) as an active ingredient is used in a method of potentiating the antitumor effect of an antitumor preparation containing 3 ingredients, i.e., tegafur, gimeracil, and oteracil potassium, as active ingredients, and the antitumor effect of this antitumor preparation can be significantly potentiated.

In the present invention, a feature of the use for treating cancer is concomitantly administering to a mammal tegafur in a therapeutically effective amount, gimeracil in an amount effective for potentiating an antitumor effect, oteracil potassium in an amount effective for inhibiting a side effect, and cis-oxalate(1R,2R-diaminocyclohexane)platinum(II) in an amount effective for potentiating an antitumor effect.

A feature of the pharmaceutical composition (antitumor preparation) of the present invention is that it is present in a form comprising a plurality of pharmaceutical agents each of which contains one active ingredient selected from tegafur, gimeracil, oteracil potassium and 1-OHP, or each of which contains such active ingredients in any combination, or in a pharmaceutical form comprising a single pharmaceutical agent containing all these active ingredients.

In the present invention, a feature of the method for potentiating an antitumor effect is administering 1-OHP in an amount effective for potentiating an antitumor effect in combination with an antitumor preparation comprising tegafur in a therapeutically effective amount, gimeracil in an amount effective for potentiating an antitumor effect, and oteracil potassium in an amount effective for inhibiting a side effect.

Cis-oxalate(1R,2R-diaminocyclohexane) platinum(II) (oxaliplatin, 1-OHP) is a platinum-containing complex, and is a known compound. 1-OHP induces functional disorder of DNA and DNA strand breakage by binding to the DNA of cancer cells, thereby exerting an action of annihilating cancer cells. 1-OHP can be produced according to known methods, for example, the method disclosed in Japanese Examined Patent Publication No. 1985-41077 .

Tegafur (generic name, chemical name: 5-fluoro-1-(2-tetrahydrofuryl)-2,4-(1H,3H)-pyrimidinedione, hereinafter sometimes referred to as FT), an active ingredient of the antitumor preparation, is a known compound, and it is a drug activated in vivo and releases an active principle, i.e., 5-FU, thereby revealing an antitumor activity. Tegafur can be produced according to known methods, for example, the method disclosed in Japanese Examined Patent Publication No. 1974-10510.

Gimeracil (generic name, chemical name: 2,4-dihydroxy-5-chloropyridine, hereinafter sometimes referred to as CDHP) is also a known compound, and although it does not exhibit any antitumor activity by itself, it can potentiate an antitumor effect by inhibiting the in vivo metabolic inactivation of 5-FU.

Oteracil potassium (generic name, chemical name: monopotassium 1,2,3,4-tetrahydro-2,4-dioxo-1,3,5-triazine-6-carboxylate, hereinafter sometimes referred to as OXO) is also a known compound. Although it does not exhibit any antitumor activity by itself, it mostly remains in the gastrointestinal tract and inhibits the activation of 5-FU at that location, thereby preventing gastrointestinal tract disorders caused by 5-FU.

With respect to the antitumor preparation containing 3 ingredients, i.e., tegafur, gimeracil, and oteracil potassium, as active ingredients, the proportion of each active ingredient may be within the ranges described in connection with a known compound pharmaceutical agent, for example, that disclosed in Patent Publication No. 2614164 . It is usually such that, per mole of tegafur, gimeracil is used in a proportion of about 0.1-5 mole and preferably about 0.2-1.5 mole, and oteracil potassium is used in a proportion of about 0.1-5 mole and preferably about 0.2-2 mole. A particularly preferable proportion of the 3 ingredients is tegafur : gimeracil : oteracil potassium = 1:0.4:1 in molar ratio (hereinafter, a compound pharmaceutical agent containing the ingredients in this ratio is sometimes referred to as TS-1).

The antitumor preparation containing tegafur, gimeracil, and oteracil potassium as active ingredients may be prepared in a pharmaceutical form comprising two or more pharmaceutical agents each of which contains one of the active ingredients, or each of which contains such active ingredients in any combination, or in a pharmaceutical form comprising a single pharmaceutical agent containing all of the active ingredients. In either case, such antitumor preparations are prepared as pharmaceutical compositions according to standard methods using suitable pharmaceutical carriers. Carriers usable herein are those that are commonly used in conventional drugs, for example, excipients, binders, disintegrators, lubricants, colorants, taste enhancers, flavor enhancers, surfactants, etc.

When an antitumor preparation in a pharmaceutical form comprising two or more pharmaceutical agents as described above is used, each pharmaceutical agent may be administered concurrently, or one pharmaceutical agent may be administered any time before or after the administration of the other pharmaceutical agent(s). Preferably, all of the pharmaceutical agents are administered concurrently, or one pharmaceutical agent is administered within 4 hours, and more preferably within 2 hours, before or after the administration of the other pharmaceutical agent(s).

The 1-OHP as an active ingredient for use in a method of potentiating an antitumor effect of the present invention (hereinafter also referred to as the *antitumor effect potentiator*) may be prepared singly in a unit dosage form. In this case, the antitumor effect potentiator is prepared as a pharmaceutical composition according to standard methods using suitable pharmaceutical carriers. Carriers usable herein are those that are commonly used in conventional drugs, for example, excipients, binders, disintegrators, lubricants, colorants, taste enhancers, flavor enhancers, surfactants, etc. The antitumor effect potentiator prepared in any unit dosage form may be administered concurrently with, or before or after, the antitumor preparation containing 3 ingredients, i.e., tegafur, gimeracil, and oteracil potassium, as active ingredients that may also be prepared in any unit dosage form. That is, the antitumor effect potentiator of the present invention can be administered any time before or after or concurrently with the administration of the antitumor preparation containing 3 ingredients, i.e., tegafur, gimeracil, and oteracil potassium, as active ingredients.

Preferably, the antitumor effect potentiator is administered concurrently with or within 4 hours before or after the administration of the antitumor preparation, and preferably within 2 hours, before or after the administration of the antitumor preparation.

When the antitumor effect potentiator is administered concurrently with, or before or after, the aforementioned antitumor preparation containing 3 ingredients, i.e., tegafur, gimeracil, and oteracil potassium, as active ingredients, the antitumor effect potentiator is preferably administered in an amount such that the amount of 1-OHP, per mole of tegafur, is within the range of about 0.1 to about 5 mole, preferably about 0.3 to about 3 mole, and more preferably about 0.4 to about 1 mole.

Unit dosage forms usable for administering the antitumor effect potentiator to treat malignant tumors of mammals, including humans, are not limited, and can be suitably selected according to the purpose of the treatment. Specific examples are injections, suppositories, ophthalmic solutions, ointments, aerosols, and like parenteral forms; tablets, coated tablets, powders, granules, capsules, fluids, pills, suspensions, emulsions, and like oral forms, with injections being of a preferable form of administration. The antitumor effect potentiator can be produced in such dosage forms according to methods commonly known in this technical field.

According to the present invention, a pharmaceutical composition (antitumor preparation) containing an antitumor effect potentiator can be prepared, in which 1-OHP, which is the active ingredient of the aforementioned antitumor effect potentiator, is concomitantly present with an antitumor preparation containing 3 ingredients, i.e. tegafur, gimeracil, and oteracil potassium, as active ingredients. Such an antitumor preparation may be in a pharmaceutical form comprising a plurality of pharmaceutical agents each of which contains one of the aforementioned 4 ingredients, or each of which contains such ingredients in any combination, or in a pharmaceutical form comprising a single pharmaceutical agent containing all of the ingredients. In other words, such an antitumor preparation may be a drug comprising a single portion consisting of a pharmaceutical agent containing all of the aforementioned 4 ingredients, or may be a drug comprising multiple portions consisting of a pharmaceutical agent containing 1 to 3 ingredients and a pharmaceutical agent containing another ingredient. In particular, a 2-part preparation in which a compound pharmaceutical agent containing 3 ingredients, i.e., tegafur, gimeracil, and oteracil potassium, as active ingredients and a pharmaceutical agent containing 1-OHP as an active ingredient are present as separate portions is preferable.

With respect to the antitumor preparation, the proportion of the ingredients is not limited, irrespective of being composed of a single pharmaceutical agent or multiple pharmaceutical agents. Usually, per mole of tegafur, gimeracil is used in a proportion of about 0.1-5 mol, preferably about 0.2-1.5 mol, oteracil potassium is used in a proportion of about 0.1-5 mol, preferably about 0.2-2 mol, and 1-OHP is used in a proportion of about 0.1-5 mol, preferably about 0.3-3 mol, and more preferably about 0.4-1 mol. In particular, a preferable molar ratio of the ingredients is tegafur : gimeracil : oteracil potassium : 1-OHP = about 1 :0.4:1: (0.1-5), more preferably about 1:0.4:1:(0.3-3), and particularly preferably about 1:0.4:1:(0.4-1). When the antitumor preparation is a 2-part drug in which a compound pharmaceutical agent containing 3 ingredients, i.e., tegafur, gimeracil, and oteracil potassium, as active ingredients and a pharmaceutical agent containing 1-OHP as an active ingredient are present as separate portions as described above, the antitumor preparation preferably contains the compound pharmaceutical agent containing tegafur, gimeracil, and oteracil potassium in a molar ratio of 1 : 0.4 : 1, and the pharmaceutical preparation containing, per mole of tegafur, 1-OHP in a proportion of about 0.1-5 mol, preferably about 0.3-3 mol, and more preferably about 0.4-1 mol.

The active ingredients may be prepared as pharmaceutical compositions according to standard methods using suitable pharmaceutical carriers. Carriers usable herein are those that are commonly used in conventional drugs, for example, excipients, binders, disintegrators, lubricants, colorants, taste enhancers, flavor enhancers, surfactants, etc.

When a multi-part antitumor preparation comprising two or more pharmaceutical agents as described above is used, each pharmaceutical agent may be administered concurrently, or each pharmaceutical agent may be administered any time before or after the administration of the other pharmaceutical agent(s). Preferably, all of the pharmaceutical agents are administered concurrently, or one pharmaceutical agent is administered within 4 hours, and more preferably within 2 hours, before or after the administration of the other pharmaceutical agents.

The pharmaceutical composition of the present invention comprises

(a) a pharmaceutical agent (antitumor composition) containing

(i) tegafur in a therapeutically effective amount,

(ii) gimeracil in an amount effective for potentiating an antitumor effect, and

(iii) oteracil potassium in an amount effective for inhibiting a side effect, and

(b) a pharmaceutical agent (composition) containing 1-OHP in an amount effective for potentiating an antitumor effect.

They may be in any known pharmaceutical form. The compositions are usually housed in any commonly-used containers according to their pharmaceutical form.

For example, the pharmaceutical composition of the present invention can be present in at least 2 containers for housing the above active ingredients, in which tegafur and 1-OHP are housed in separate containers. The aforementioned ingredients (i)-(iv) are preferably in a pharmaceutical form prepared in combination with pharmaceutically acceptable carriers. Insofar as ingredients (i) and (iv) are housed in separate containers, ingredients (ii) and (iii) may be independently housed in containers that are separate from those in which the aforementioned two ingredients are housed, or ingredients (ii) and (iii) may be independently mixed with ingredient (i) or (iv) for housing in the same container. Preferably, a pharmaceutical agent containing ingredients (i)-(iii) is housed in one container and a pharmaceutical agent containing ingredient (iv) is housed in another container.

Unit dosage forms usable in administering the pharmaceutical composition of the present invention to treat malignant tumors of mammals, including humans suffering malignant tumors, are not limited, and can be selected according to the purpose of the treatment. Specific examples are parenteral forms (such as injections, suppositories, ophthalmic solutions, ointments and aerosols) and oral forms (such as tablets, coated tablets, powders, granules, capsules, fluids, pills, suspensions and emulsions). The antitumor preparation can be produced in such dosage forms according to methods commonly known in this technical field.

In connection with the antitumor effect potentiator and the pharmaceutical composition of the present invention, in preparing solid oral agents, such as tablets, powders, and granules, for example, the following can be used as carriers: excipients (such as lactose, saccharose, sodium chloride, glucose, urea, starch, calcium carbonate, kaolin, crystalline cellulose, silicic acid, methylcellulose, glycerol, sodium alginate and gum arabic); binders (such as simple syrups, liquid glucose, liquid starch, gelatin solutions, polyvinyl alcohol, polyvinyl ether, polyvinylpyrrolidone, carboxymethylcellulose, shellac, methylcellulose, ethylcellulose, water, ethanol and potassium phosphate); disintegrators (such as dried starch, sodium alginate, powdered agar, powdered laminaran, sodium hydrogencarbonate, calcium carbonate, polyoxyethylene sorbitan fatty acid esters, sodium lauryl sulfate, stearic acid monoglycerides, starch and lactose); disintegration inhibitors (such as saccharose, stearic acid, cocoa butter and hydrogenated oils); absorption enhancers (such as sodium lauryl sulfate); humectants (such as glycerol and starch); adsorbents (such as starch, lactose, kaolin, bentonite and colloidal silicic acid), and lubricants (such as purified talc, stearic acid salts, powdered boric acid and polyethylene glycol). Furthermore, tablets may be provided with a standard coating as necessary, such as sugar-coated tablets, gelatin-coated tablets, enteric-coated tablets, film-coated tablets, double-layer tablets and multilayer tablets.

In preparing pills, for example excipients (such as glucose, lactose, starch, cacao butter, hydrogenated vegetable oils, kaolin and talc), binders (such as powdered gum arabic, powdered tragacanth and gelatin) and disintegrants (such as laminaran and agar) can be used as carriers.

Capsules can be prepared by mixing the active ingredients with the aforementioned various carriers and filling e.g. hard gelatin capsules or soft capsules with the mixture.

In preparing suppositories, for example, polyethylene glycol, cacao butter, lanolin, higher alcohols, esters of higher alcohols, gelatin, semisynthetic glycerides, or Witepsol (registered trademark, Dynamite Nobel Inc.) can be used as carriers.

In preparing injections, examples of carriers that can be used are diluents (such as water, ethyl alcohol, macrogol, propylene glycol, ethoxylated isostearyl alcohol, polyoxylated isostearyl alcohol and polyoxyethylene sorbitan fatty acid esters); pH-adjusters (such as sodium citrate, sodium acetate and sodium phosphate); buffers (such as dipotassium phosphate, trisodium phosphate, sodium hydrogen phosphate and sodium citrate); stabilizers (such as sodium pyrosulfite, EDTA, thioglycolic acid and thiolactic acid); and saccharides for binders in freeze-drying (such as mannitol, inositol, maltose, sucrose and lactose).

In such cases, glucose and glycerol may be used in the pharmaceutical composition in amounts sufficient to prepare an isotonic solution. Moreover, e.g. standard auxiliary dissolvents, soothing agents and topical anesthetics may be used. Subcutaneous, intramuscular, and intravenous injections can be prepared according to standard methods in conjunction with such carriers.

Liquid preparations may take a form of water-based or oilbased suspensions, solutions, syrups, or elixirs, and can be prepared according to standard methods using commonly-used additives.

In preparing the pharmaceutical composition in a form of ointments such as pastes, creams, and gels, for example, white petrolatum, paraffin, glycerol, cellulose derivatives, polyethylene glycol, silicon or bentonite can be used as diluents.

The amounts of tegafur, gimeracil, oteracil potassium, and l-OHP, which are active ingredients of the pharmaceutical composition (antitumor preparation) of the present invention, vary according to e.g. the dosage form, route of administration or the dosing schedule, and are not limited, and hence can be suitably selected. It is usually preferable that the proportion of the active ingredients accounts for about 1-70 wt.-% of the pharmaceutical preparation.

Methods for administration of the pharmaceutical composition of the present invention are not limited and can be determined according to the form thereof, the age, sex, and condition of the patient, and other factors, and hence it may be administered, for example, enterally, orally, rectally, intraorally, intraarterially, intravenously or transdermally. For example, tablets, pills, solutions, suspensions, emulsions, granules, capsules are administered orally; injections are administered intraarterially or intravenously; suppositories are administered intrarectally; and ointments are applied to e.g. the skin or the mucous membrane in the mouth. With respect to the pharmaceutical composition of the present invention, it is possible that the compound pharmaceutical agent containing tegafur, gimeracil, and oteracil potassium is orally administered while the l-OHP containing pharmaceutical agent is intravenously administered.

The dosage of each active ingredient in the present invention can be suitably selected according to the application, the age and sex of the patient, the degree of the disease, and other factors. The antitumor effect potentiator and the pharmaceutical composition (antitumor preparation) of the present invention can be administered in 1-4 doses per day.

In oral administration, the pharmaceutical composition of the present invention is preferably given in an amount using the following ranges as a standard: the amount of tegafur is about 0.1-100 mg/kg/day, preferably about 0.2-40 mg/kg/day, and more preferably about 0.5-20 mg/kg/day; the amount of gimeracil is about 0.02-30 mg/kg/day, preferably about 0.05-12 mg/kg/day, and more preferably about 0.1-6 mg/kg/day; the amount of oteracil potassium is about 0.1-100 mg/kg/day, preferably about 0.2-40 mg/kg/day, and more preferably about 0.5-20 mg/kg/day; and the amount of 1-OHP is about 0.08-200 mg/kg/day, preferably about 0.15-80 mg/kg/day, and more preferably about 0.4-40 mg/kg/day.

When in the form of an injection, the pharmaceutical composition, which may be diluted with aqueous glucose solution if necessary, can be gradually administered to an adult over 5 minutes or longer, usually in an amount corresponding to about 0.1-100 mg/kg/day of tegafur and about 0.08-200 mg/kg/day of 1-OHP.

When in the form of a suppository, the pharmaceutical composition of the invention is administered once or twice a day at an interval of 6-12 hours usually in an amount in an adult corresponding to about 0.1-100 mg/kg/day of tegafur and about 0.08-200 mg/kg/day of l-OHP by inserting it into the rectum.

The types of malignant tumors treatable by the administration of the pharmaceutical composition of the present invention is not limited, insofar as the active principle, i.e., 5-FU, is reactive thereto; for example, head and neck cancer, stomach cancer, colon cancer, rectal cancer, liver cancer, gallbladder/biliary cancer, pancreatic cancer, lung cancer, breast cancer, vesical cancer, prostatic cancer, uterine cancer, esophageal cancer, renal cancer and ovarian cancer. In particular, a remarkable effect can be expected from the pharmaceutical preparation of the present invention toward colon cancer, rectal cancer, breast cancer, esophageal cancer, stomach cancer, and head and neck cancer. Furthermore, a remarkable effect can be expected toward typical drug-resistant tumors and tumors that are starting to be drug-resistant.

Due to the antitumor effect potentiator and the pharmaceutical composition (antitumor preparation) of the present invention, an antitumor effect can be obtained that exceeds the effect obtained by a single use of a 3-membered compound pharmaceutical agent containing known antitumor drugs, e.g., tegafur, gimeracil, and oteracil potassium, and the effect obtained by a pharmaceutical agent containing l-OHP alone, without aggravating toxicity (gastrointestinal toxicity and bone marrow toxicity, in particular). Moreover, this antitumor effect is superior to the antitumor effect obtained by a combination therapy using a tegafur/uracil compound pharmaceutical agent and a salt of d,l-folinic acid, which is a standard therapy for cancer of the large intestine, and that obtained by a combination therapy using a tegafur/uracil compound pharmaceutical agent, a salt of d,l-folinic acid, and l-OHP. Furthermore, a remarkable antitumor effect potentiating action and antitumor effect can be expected from the pharmaceutical composition of the present invention toward tumors that are resistant to 5-FU or other various drugs.

BEST MODE FOR CARRYING OUT THE INVENTION

Examples are given below to illustrate the invention in more detail.

Pharmacological Test Example 1

Formulation of pharmaceutical preparations

A tegafur/gimeracil/oteracil potassium compound pharmaceutical agent (tegafur/gimeracil/oteracil potassium molar ratio = 1 : 0.4 : 1, hereinafter sometimes referred to as TS-1), a tegafur/uracil compound pharmaceutical agent (tegafur/uracil molar ratio = 1 : 4, hereinafter sometimes referred to as UFT), and *d,l*-calcium folinate were independently dissolved or suspended in 0.5% hydroxypropylmethyl cellulose (HPMC) solutions, and pharmaceutical preparations were formulated so as to allow administration thereof in a final dosage of 10 mL/kg. For example, if tegafur was to be administered in an amount of 8.3 mg/kg, a pharmaceutical agent corresponding to 8.3 mg of tegafur was dissolved or suspended in 10 mL of HPMC, and an oral pharmaceutical preparation was then formulated such that the drug could be administered in an amount of 10 mL/kg.

l-OHP was dissolved in 5% aqueous glucose solution (Otsuka Glucose, manufactured by Otsuka Pharmaceutical Factory Inc.), thereby giving an l-OHP preparation.

Administration of pharmaceutical preparations

A fragment measuring about 2 mm cubic fragment of the human colon cancer xenograft KM20C strain was subcutaneously implanted on the backs of male nude rats F344/NJcl-*rnu*. When the average tumor volume (= 0.5 x major axis (mm) x minor axis $(mm)^2$) reached about 200 $mm^3$, the rats were divided into groups (day 0).

The TS-1 preparation or the UFT+LV preparation prepared above was orally administered once a day in amounts presented in Table 1 from the day after grouping (day 1) for 14 consecutive days. The l-OHP preparation was administered into the tail vein on day 1 at 10 mg/kg immediately before the administration of the aforementioned preparations.

The ratio of the tumor volume upon grouping (day 0) to the tumor volume on day 15 was regarded as the relative tumor volume: Tumor volume on day 15 / Tumor volume on day 0

The extent of tumor growth inhibition (%) was calculated using the average relative tumor volume of the drug-administered groups and the average relative tumor volume of the control group:

$$[1 - \text{(Relative tumor volume of drug-administered group)} / \text{(Relative tumor volume of tumor-bearing control group)}] \times 100 \ (\%)$$

Moreover, the effect due to the combined use was analyzed using the relative tumor volumes on day 15 according to the IUT procedure (Intersection-Union test) (see Statistical Science 1996, Vol. 11, No. 4, 283-319).

The difference in body weight between rats on day 0 and rats on day 15 presented as a ratio (extent of body weight change) was used as an index of systemic toxicity of the pharmaceutical preparation:

$$[(\text{Body weight of rats on day 15} - \text{Body weight of rats on day 0}) / \text{body weight of rats on day 0}] \times 100\ (\%)$$

The results thus obtained are presented on Table 1. Although calcium folinate was used in the experiment, the dosage given in the table is that calculated as the equivalent amount of folinic acid.

Table 1

| Drug | Dosage of compound agent containing tegafur (mg/kg /day) | Dosage of oxaliplatin (mg/kg/day) | Relative tumor volume (Mean) | Extent of tumor growth inhibition (%) | Extent of body weight change (%) |
|---|---|---|---|---|---|
| None | - | - | 5.81 | - | 1.3 |
| Oxaliplatin (1-OHP) | - | 10 | 4.64 | 20.1 | 0.9 |
| Tegafur + gimeracil + oteracil potassium (TS-1) | 12 + 3.5 + 11.7 | - | 2.83 | 51.4 | -3.4 |
| Tegafur + gimeracil + oteracil potassium + oxaliplatin (TS-1 + 1-OHP) | 12 + 3.5 + 11.7 | 10 | 2.13 *,## | 63.3 | -6.4 |
| Tegafur + uracil + folinic acid (UFT + LV) | 24 + 53.8 + 20.0 | - | 3.73 | 35.8 | -3.1 |
| Tegafur + uracil + folinic acid + oxaliplatin (UFT + LV + 1-OHP) | 24 + 53.8 + 20.0 | 10 | 2.79 * | 51.9 | -4.5 |

*: Group with a significant effect determined according to the IUT procedure ($p<0.05$)
##: Group with a significant effect compared to a group given UFT + LV in combination with the same amount of 1-OHP ($p<0.01$)

The results presented above establish that the administration of TS-1 in combination with l-OHP is an extremely effective therapeutic method since it provides statistically significant effects when compared with the administration of TS-1 alone, and, when compared with the administration of UFT+LV in combination with l-OHP, affords statistically superior effects without substantially increasing toxicity.

Pharmacological Test Example 2 (Action toward a 5-FU-resistant strain)

A fragment measuring about 2 mm cubic fragment of a 5-FU-resistant strain of the human colon cancer xenograft KM12C strain established by the applicant was subcutaneously implanted on the backs of male nude mice BALB/c-nu/nu. When the average tumor volume (= 0.5 x major axis (mm) x minor axis (mm)$^2$) reached about 200 mm$^3$, the mice

were divided into groups (day 0). The TS-1 preparation formulated in Pharmacological Test Example 1 was orally administered to the mice once a day in an amount as shown in Table 2 from day 1 for 9 consecutive days. The l-OHP preparation was administered into the tail vein on day 1 at 8.3 mg/kg immediately before the administration of the TS-1 preparation. The ratio of the tumor volume on day 0 to the tumor volume on day 10 was calculated to obtain a relative tumor volume. As in Pharmacological Test Example 1, the extent of tumor growth inhibition and the extent of body weight change were calculated using the average relative tumor volumes of the drug-administered groups and the control group.

Table 2

| Drug | Dosage of tegafur-containing agent (mg/kg/day) | Dosage of oxaliplatin-containing agent (mg / kg / day) | Relative tumor volume (Mean) | Extent of tumor growth inhibition (%) | Extent of body weight chance (%) |
|---|---|---|---|---|---|
| None | - | - | 9.5 | - | -6.2 |
| Oxaliplatin (1-OHP) | - | 8.3 | 7.97 | 16.1 | -8.0 |
| Tegafur + gimeracil + oteracil potassium (TS-1) | 8.3 + 2.4 + 8.1 | - | 6.39 | 32.7 | -9.2 |
| Tegafur + gimeracil + oteracil potassium + oxaliplatin (TS-1 + 1-OHP) | 8.3 + 2.4 + 8.1 | 8.3 | 4.89 * | 48.5 | -18.6 |
| *: Group with a significant effect determined according to the IUT procedure ($p<0.05$) | | | | | |

The results presented above establish that the administration of TS-1 in combination with l-OHP is an effective therapeutic method toward 5-FU-resistant strains since, while TS-1 alone barely exhibits any effect on the 5-FU-resistant strain, the administration of TS-1 in combination with l-OHP potentiated the extent of tumor growth inhibition to essentially the same extent (about 50%) as with the 5-FU-sensitive strains, without accompanied by a significant body weight change.

Pharmacological Test Example 3 (Action toward a multidrug-resistant strain)

A fragment measuring about 2 mm cubic fragment of the human colon cancer xenograft HCT-15 strain, which expresses P-glycoprotein in large amounts and is resistant to multiple drugs, was subcutaneously implanted on the backs of male nude mice BALB/*c-nu/nu*. When the average tumor volume (= 0.5 x major axis (mm) x minor axis (mm)$^2$) reached about 200 mm$^3$, the mice were divided into groups (day 0). A TS-1 preparation as prepared in Pharmacological Test Example 1 was orally administered to the mice once a day in an amount as shown in Table 3 from day 1 for 14 consecutive days. The l-OHP preparation was administered only once into the tail vein on day 1 at 10.0 mg/kg immediately before the administration of the TS-1 preparation. The extent of tumor growth inhibition was calculated as in Pharmacological Test Example 1. Using the relative tumor volumes on day 15, the difference in relative tumor volume between the control group and the drug-administered groups was determined according to a Dunnett's test, and the difference between the one which was given either TS-1 or l-OHP and the one which was given TS-1 and l-OHP in combination was evaluated according to Student's t-test.

Table 3

| Drug | Dosage of compound agent containing tegafur (mg/kg/day) | Dosage of oxaliplatin (mg/kg/day) | Relative tumor volume (Mean) | Extent of tumor growth inhibition (%) |
|---|---|---|---|---|
| None | - | - | 4.53 | - |
| Oxaliplatin (1-OHP) | - | 10 | 4.33 | 4.3 |
| Tegafur + gimeracil + oteracil potassium (TS-1) | 10 + 2.9 + 9.8 | - | 2.56 * | 43.4 |
| Tegafur + gimeracil + oteracil potassium + oxaliplatin (TS-1 + 1-OHP) | 10 + 2.9 + 9.8 | 10 | 1.54**,#,$ | 66.0 |

*: Group with a significant effect relative to the tumor-bearing control group ($p<0.05$)
**: Group with a significant effect relative to the tumor-bearing control group ($p<0.01$)
\#: Group with a significant effect relative to the one which was given TS-1 alone ($p<0.025$)
$: Group with a significant effect relative to the one which was given oxaliplatin alone ($p<0.01$)

As demonstrated above, the administration of TS-1 in combination with l-OHP significantly potentiated the antitumor effect of TS-1 on a tumor on which l-OHP alone barely shows any antitumor effect. The results therefore establish that the potentiation of antitumor effect is due to the antitumor effect potentiating activity of l-OHP toward TS-1. The results suggest that a combination therapy using TS-1 and l-OHP is effective against tumors that are insensitive to a large number of anticancer preparations since the tumor used herein is a multidrug-resistant tumor.

Pharmacological Test Example 4 (Dose dependency of l-OHP)

A fragment measuring about 2 mm cubic fragment of the human colon cancer xenograft COL-1 strain was subcutaneously implanted on the backs of male nude mice BALB/*c-nu/nu*. When the average tumor volume (= 0.5 x major axis (mm) x minor axis $(mm)^2$) reached about 125 $mm^3$, the mice were divided into groups (day 0). The TS-1 preparation formulated in Pharmacological Test Example 1 was orally administered to the mice once a day in an amount equivalent to 6.9 mg/kg of tegafur from the day after grouping for 14 consecutive days. l-OHP dissolved in 5% aqueous glucose solution was administered into the tail vein on days 1 and 8 at 2.8, 3.5, 4.2, or 5.0 mg/kg/day immediately before the administration of the TS-1 preparation.

For the determination of antitumor effect, the ratio of the tumor volume upon grouping (day 0) to the tumor volume on day 15 was calculated to obtain a relative tumor volume. The extent of tumor growth inhibition was determined using the average relative tumor volumes of the drug-administered groups and the control group. Using the relative tumor volumes on the evaluation day, the statistical significance of the difference in relative tumor volume between the control group and the one which was given the TS-1 preparation alone was analyzed according to Student's t-test; the correlation between the antitumor effect by l-OHP and the dosage thereof was analyzed according to a Williams' test; the statistical significance of the difference in relative tumor volume between the control group and the one which was given l-OHP alone was analyzed according to a Dunnett's test; and the effect observed in the one which was given TS-1 and l-OHP in combination relative to the one which was given either TS-1 or l-OHP was analyzed according to the IUT procedure.

The extent of body weight change between the mice on day 0 and the mice on day 15 was used as an index of systemic toxicity of l-OHP.

Table 4

| Drug | Dosage of compound agent containing tegafur (mg/kg) | Dosage of oxaliplatin (mg/kg) | Relative tumor volume (Mean) | Extent of tumor growth inhibition (%) | Extent of body weight change (%) | Number of dead animal |
|---|---|---|---|---|---|---|
| None | - | - | 5.75 | - | -16.1 | - |
| Tegafur + gimeracil + oteracil potassium (TS-1) | 6.9 + 2.0 + 6.8 | - | 3.80** | 33.9 | -20.5 | 0 |
| Oxaliplatin (1-OHP) | - | 2.9 | 5.06§ | 11.9 | -18.0 | 0 |
| 1-OHP | - | 3.5 | 4.54 §,* | 21.0 | -20.6 | 0 |
| 1-OHP | - | 4.2 | 4.43§,** | 23.0 | -19.0 | 0 |
| 1-OHP | - | 5.0 | 4.41§,** | 23.3 | -16.8 | 0 |
| TS-1+1-OHP | 6.9 + 2.0 + 6.8 | 2.9 | 3.70# | 35.7 | -20.1 | 0 |
| TS-1+1-OHP | 6.9 + 2.0 + 6.8 | 3.5 | 2.55# | 55.6 | -19.3 | 0 |
| TS-1+1-OHP | 6.9 + 2.0 + 6.8 | 4.2 | 2.65 # | 53.9 | -22.0 | 0 |
| TS-1+1-OHP | 6.9 + 2.0 + 6.8 | 5.0 | 2.34 # | 59.3 | -23.4 | 1 |

*: Group with a significant effect relative to the control group ($p<0.05$)
**: Group with a significant effect relative to the control group ($p<0.01$)
§: Group with an effect significantly correlated with dosage ($p<0.05$)
#: Group with a significant effect by a combined use determined according to the IUT procedure ($p<0.05$)

The results given above establish that the effect of the tegafur/gimeracil/oteracil potassium compound pharmaceutical agent is proportional to the dosage of l-OHP.

Pharmacological Test Example 5 (Test for efficacy comparison) Preliminary Test (Investigation of maximum tolerable dose of capecitabine)

A fragment measuring about 2 mm cubic fragment of the human colon cancer xenograft COL-1 strain was subcutaneously implanted on the backs of male nude mice BALB/c-nu/nu. When the average tumor volume (= 0.5 x major axis (mm) x minor axis (mm)$^2$) reached about 190 mm$^3$, the mice were divided into groups (day 0). Capecitabine suspended in 0.5% HPMC solution was orally administered to the mice once a day at 240, 360, or 540 mg/kg from the day after grouping for 14 consecutive days. l-OHP dissolved in 5% aqueous glucose solution was administered into the tail vein on days 1 and 8 at 4.2 mg/kg/day immediately before the administration of the Capecitabine. The difference in body weight between the mice on day 0 and the mice on day 15 presented as a ratio and the occurrence of death were used as indices of systemic toxicity of capecitabine. The results showed that the maximum tolerable dose at which death due to toxicity is not observed of capecitabine is 360 mg/kg/day.

Main Test (Test for efficacy comparison)

A fragment measuring about 2 mm cubic fragment of the human colon cancer xenograft COL-1 strain was subcutaneously implanted on the backs of male nude mice BALB/c-nu/nu. When the average tumor volume (= 0.5 x major axis (mm) x minor axis (mm)$^2$) reached about 170 mm$^3$, the mice were divided into groups (day 0). The TS-1 preparation formulated in Pharmacological Test Example 1 was orally administered to a group of mice once a day from the day after grouping for 14 consecutive days in an amount equivalent to the maximum tolerable dose of tegafur, i.e., 6.9 mg/kg. l-OHP dissolved in 5% aqueous glucose solution was administered into the tail vein on days 1 and 8 in the maximum tolerable dose, i.e., 4.2 mg/kg/day immediately before the administration of the TS-1 preparation. Capecitabine suspended in 0.5% HPMC solution was orally administered to another group of mice once a day from the day after grouping for 14 consecutive days in the maximum tolerable amount determined in the preliminary test, i.e., 360 mg/kg. l-OHP dissolved in 5% aqueous glucose solution was administered into the tail vein on days 1 and 8 at 4.2 mg/kg/day immediately before the administration of the Capecitabine.

For the determination of antitumor effect, the ratio of the tumor volume upon grouping (day 0) to the tumor volume on day 15 was calculated to obtain a relative tumor volume. The extent of tumor growth inhibition was determined using the average relative tumor volumes of the drug-administered groups and the control group. Using the relative tumor volumes on the test day, the statistical significance of the difference in relative tumor volume between the control group and the one which was given the pharmaceutical agents was analyzed according to a Dunnett's test; and the statistical significance of the difference in relative tumor volume between the one which was given TS-1 and l-OHP in combination and the one which was given capecitabine and l-OHP in combination was analyzed according to Student's t-test. The extent of body weight change between the mice on day 0 and the mice on day 15 was used as an index of systemic toxicity of the pharmaceutical preparations.

Table 5

| Drug | Dosage of fluorinated pyrimidine (mg/kg) | Dosage of oxaliplatin (mg/kg) | Time of oxaliplatin administration (day) | Relative tumor volume (Mean) | Extent of tumor growth inhibition (%) | Extent of body weight change (%) |
|---|---|---|---|---|---|---|
| None | - | - | - | 5.42 | - | -14.8 |
| Tegafur + gimeracil + oteracil potassium+oxaliplatin (TS-1+1-OHP) | 6.9 + 2.0 + 6.8 | 4.2 | 1,8 | 1.87 ***,### | 65.5 | -24.0 |
| Capecitabine + oxaliplatin | 360 | 4.2 | 1,8 | 2.94 *** | 45.7 | -26.4 |
| ***: Group with a significant effect relative to the control group ($p<0.001$) ###: Group with a significant effect relative to the one which was given capecitabine + oxaliplatin ($p<0.001$) | | | | | | |

The test for efficacy comparison at maximum tolerable doses described above demonstrated that a combination therapy using a tegafur/gimeracil/oteracil potassium compound pharmaceutical agent and l-OHP shows a clearly better antitumor effect than a combination therapy using capecitabine and l-OHP. The systemic toxicities (body weight losses) were almost identical.

The results of the Test Examples presented above reveal that a combination therapy using a tegafur/gimeracil/ oteracil potassium compound pharmaceutical agent and l-OHP significantly potentiates antitumor activity without substantially aggravating side effects compared with the use of a tegafur/gimeracil/oteracil potassium compound pharmaceutical agent alone. The results also demonstrate that, in terms of antitumor effect, the combination therapy is a significantly more effective therapeutic method compared with a standard therapy in which a tegafur/uracil compound pharmaceutical agent and d,l-calcium folinate as well as capecitabine are used in combination. Although it has been considered that, in the case of cancers recurring after therapies mainly using fluorinated pyrimidines, repeating such therapies in which fluorinated pyrimidines are mainly used are likely to be fruitless, the results of Pharmacological Test Example 2 establish that a tegafur/gimeracil/oteracil potassium compound pharmaceutical agent can be advantageously and continuously used in such treatments without substantially aggravating side effects if used in combination with l-OHP. Furthermore, it is herein indicated that potentiation in antitumor effect attained when l-OHP is used in combination is due to the antitumor effect potentiating action of l-OHP toward the tegafur/gimeracil/oteracil potassium compound pharmaceutical agent since a significant antitumor effect was obtained when a tegafur/gimeracil/oteracil potassium compound pharmaceutical agent was used in combination with l-OHP, even on a tumor for which l-OHP by itself does not show any antitumor effect. The results of the test using a multidrug-resistant tumor establish that a combination therapy with a tegafur/gimeracil/oteracil potassium compound pharmaceutical agent and l-OHP is effective toward tumors that are not sensitive (i.e., that are resistant) to a large number of anticancer agents.

EP 1 757 283 B1

Formulation Examples

Formulation examples of the antimumor effect potentiator and the antitumor preparation of the present invention are given below.

Formulation Example 1: Injectable solution

| 1-OHP | 100 mg |
|---|---|
| 5% Aqueous glucose solution | 50 mL |
| Amount per ampule | 50 mL |

Injectable solutions were prepared according to a standard method using the formulation presented above.

Formulation Example 2: Granule

| Tegafur | 50 mg |
|---|---|
| Gimeracil | 14.5 mg |
| Oteracil potassium | 49 mg |
| 1-OHP | 55 mg |
| Lactose | 280 mg |
| Cornstarch | 298 mg |
| Hydroxypropylmethyl cellulose | 10 mg |
| Amount per wrapper | 756.5 mg |

Granules were prepared according to a standard method using the formulation presented above.

Formulation Example 3: Capsule

| Tegafur | 25 mg |
|---|---|
| Gimeracil | 7.25 mg |
| Oteracil potassium | 24.5 mg |
| 1-OHP | 40 mg |
| Lactose | 51 mg |
| Crystalline cellulose | 28 mg |
| Magnesium stearate | 5 mg |
| Amount per capsule | 180.75 mg |

Capsules were prepared according to a standard method using the formulation presented above.

Formulation Example 4: Tablet

| Tegafur | 20 mg |
|---|---|
| Gimeracil | 5.8 mg |
| Oteracil potassium | 19.6 mg |
| 1-OHP | 39.6 mg |

(continued)

| | |
|---|---|
| Lactose | 51 mg |
| Crystalline cellulose | 15 mg |
| Magnesium stearate | 3 mg |
| Cornstarch | 14 mg |
| Hydroxypropylmethyl cellulose | 10 mg |
| Amount per tablet | 178.0 mg |

Tablets were prepared according to a standard method using the formulation presented above.

Formulation Example 5: Suppository

| | |
|---|---|
| Tegafur | 200 mg |
| Gimeracil | 58 mg |
| Oteracil potassium | 196 mg |
| 1-OHP | 396 mg |
| Witepsol W-35 | 1150 mg |
| Amount per suppository | 2000 mg |

Suppositories were prepared according to a standard method using the formulation presented above.

**Claims**

1.  Cis-oxalate(1*R*,2*R*-diaminocyclohexane) platinum(II) for use in a method of potentiating the antitumor activity of a composition comprising

    - a therapeutically effective amount of tegafur,
    - an antitumor effect potentiating amount of gimeracil and
    - a side effect inhibiting effective amount of oteracil potassium.

2.  The cis-oxalate(1*R*,2*R*-diaminocyclohexane) platinum(II) for use of claim 1, wherein the composition having antitumor activity comprises tegafur, gimeracil and oteracil potassium in a molar ratio of 1:0.4:1.

3.  Cis-oxalate(1*R*,2*R*-diaminocyclohexane) platinum(II) in combination with

    - a therapeutically effective amount of tegafur,
    - an antitumor effect potentiating amount of gimeracil and
    - a side effect inhibiting effective amount of oteracil potassium

    for use in a method of treating cancer.

4.  The cis-oxalate(*1R*,*2R*-diaminocyclohexane)platinum(II) for use of claim 3, wherein the molar ratio of cis-oxalate (1*R*,2*R*-diamino cyclohexane)platinum(II), tegafur, gimeracil and oteracil potassium is (0.1-5):1:(0.1-5):(0.1-5).

5.  The cis-oxalate(1*R*,2*R*-diaminocyclohexane) platinum(II) for use of claim 4, wherein the molar ratio is (0.1-5):1:0.4:1.

6.  The cis-oxalate(1*R*,2*R*-diaminocyclohexane) platinum(II) for use of any of claims 1-5, wherein the method comprises administering cis-oxalate(*1R,2R*-diaminocyclohexane)platinum(II) concurrently with, or within 4 hours before or after, the administration of tegafur, gimeracil and oteracil potassium.

7.  Pharmaceutical composition comprising tegafur, gimeracil, oteracil potassium, and cis-oxalate(*1R*,*2R*-diaminocyclohexane)platinum(II) as active ingredients in one or more pharmaceutical agent(s) each containing one or more of these active ingredients.

**8.** The pharmaceutical composition of claim 7, comprising

- a pharmaceutical agent comprising tegafur, gimeracil, and oteracil potassium, and
- a pharmaceutical agent comprising cis-oxalate (1*R*,2*R*-diaminocyclohexane)platinum(II).

**9.** The pharmaceutical composition of claim 7 or 8,wherein cis-oxalate(1*R*,2*R*-diaminocyclohexane)platinum(II), tegafur, gimeracil and oteracil potassium are present in a molar ratio of (0.1-5):1:(0.1-5):(0.1-5).

**10.** The pharmaceutical composition of claim 9, wherein the molar ratio is (0.1-5):1:0.4:1.

**Patentansprüche**

**1.** Cis-Oxalat(1R,2R-diaminocyclohexan)-Platin (II) zur Verwendung in einem Verfahren zur Verstärkung der Antitumoraktivität einer Zusammensetzung, umfassend

- eine therapeutisch wirksame Menge Tegafur,
- eine den Antitumoreffekt verstärkende Menge von Gimeracil und
- eine zur Inhibierung von Nebenwirkungen effektive Menge Oteracil Kalium.

**2.** Cis-Oxalat(1R,2R-diaminocyclohexan)-Platin (II) zur Verwendung gemäß Anspruch 1, wobei die Zusammensetzung mit Antitumoraktivität Tegafur, Gimeracil und Oteracil Kalium in einem molaren Verhältnis von 1:0,4:1 umfasst.

**3.** Cis-Oxalat(1R,2R-diaminocyclohexan)-Platin (II) in Kombination mit

- einer therapeutisch wirksamen Menge Tegafur,
- einer den Antitumoreffekt verstärkenden Menge Gimeracil und
- einer zur Inhibierung von Nebenwirkungen effektive Menge Oteracil Kalium

zur Verwendung in einem Verfahren zur Behandlung von Krebs.

**4.** Cis-Oxalat(1R,2R-diaminocyclohexan)-Platin (II) zur Verwendung gemäß Anspruch 3, worin das molare Verhältnis von cis-Oxalat(1R,2R-diaminocyclohexan)-Platin (II), Tegafur, Gimeracil und Oceteracil Kalium (0,1-5):1:(0,1-5):(0,1-5) beträgt.

**5.** Cis-Oxalat(1R,2R-diaminocyclohexan)-Platin (II) zur Verwendung gemäß Anspruch 4, worin das molare Verhältnis (0,1-5):1:0,4:1 beträgt.

**6.** Cis-Oxalat(1R,2R-diaminocyclohexan)-Platin (II) zur Verwendung gemäß irgendeinem der Ansprüche 1 bis 5, worin das Verfahren die Verabreichung von cis-Oxalat(1R,2R-diaminocyclohexan)-Platin (II) gleichzeitig mit oder innerhalb von 4 Stunden vor oder nach der Verabreichung von Tegafur, Gimeracil und Oteracilkalium umfasst.

**7.** Pharmazeutische Zusammensetzung, umfassend Tegafur, Gimeracil, Oteracil Kalium und cis-Oxalat(1R,2R-diaminocyclohexan)-Platin (II) als Wirkstoffe in einem oder mehreren pharmazeutischen Mittel(n), das/die jeweils eines oder mehrere dieser Wirkstoffe enthält/enthalten.

**8.** Pharmazeutische Zusammensetzung gemäß Anspruch 7, umfassend

- ein pharmazeutisches Mittel, umfassend Tegafur, Gimeracil und Oteracil Kalium, und
- ein pharmazeutisches Mittel, umfassend cis-Oxalat(1R,2R-diaminocyclohexan)-Platin (II).

**9.** Pharmazeutische Zusammensetzung gemäß Anspruch 7 oder 8, worin Cis-Oxalat(1R,2R-diaminocyclohexan)-Platin (II), Tegafur, Gimeracil und Oteracil Kalium in einem molaren Verhältnis von (0,1-5):1:(0,1-5):(0,1-5) vorliegen.

**10.** Pharmazeutische Zusammensetzung gemäß Anspruch 9, worin das molare Verhältnis (0,1-5):1:0,4:1 beträgt.

**Revendications**

1. Cis-oxalate (1R,2R-diaminocyclohexane) platine (II) pour son utilisation dans une méthode de potentialisation de l'activité antitumorale d'une composition comprenant

   - une quantité thérapeutiquement efficace de tégafur,
   - une quantité de giméracil potentialisant l'effet antitumoral et
   - une quantité efficace d'otéracil de potassium inhibant les effets secondaires.

2. Cis-oxalate (1R,2R-diaminocyclohexane) platine (II) pour son utilisation selon la revendication 1, dans lequel la composition ayant une activité antitumorale comprend le tégafur, le giméracil et l'otéracil de potassium dans un rapport molaire de 1 : 0,4 : 1.

3. Cis-oxalate (1R,2R-diaminocyclohexane) platine (II) en combinaison avec

   - une quantité thérapeutiquement efficace de tégafur,
   - une quantité de giméracil potentialisant l'effet antitumoral et,
   - une quantité efficace d'otéracil de potassium inhibant les effets secondaires,

   pour son utilisation dans une méthode de traitement du cancer.

4. Cis-oxalate (1R,2R-diaminocyclohexane) platine (II) pour son utilisation selon la revendication 3, dans lequel le rapport molaire du cis-oxalate (1R,2R-diaminocyclohexane) platine (II), du tégafur, du giméracil et de l'otéracil de potassium est de (0,1 - 5) : 1 : (0,1 - 5) : (0, 1 - 5).

5. Cis-oxalate (1R,2R-diaminocyclohexane) platine (II) pour son utilisation selon la revendication 4, dans lequel le rapport molaire est de (0,1 - 5) : 1 : 0,4 : 1.

6. Cis-oxalate (1R,2R-diaminocyclohexane) platine (II) pour son utilisation selon l'une quelconque des revendications 1 à 5, dans lequel la méthode comprend l'administration du cis-oxalate (1R,2R-diaminocyclohexane) platine (II) simultanément à, ou dans les 4 heures avant ou après, l'administration de tégafur, de giméracil et d'otéracil de potassium.

7. Composition pharmaceutique comprenant du tégafur, du giméracil, de l'otéracil de potassium, et du cis-oxalate (1R, 2R-diaminocyclohexane) platine (II) comme principes actifs dans un ou plusieurs agent(s) pharmaceutique(s) contenant chacun un ou plusieurs de ces principes actifs.

8. Composition pharmaceutique selon la revendication 7, comprenant

   - un agent pharmaceutique comprenant le tégafur, le giméracil, et l'otéracil de potassium, et
   - un agent pharmaceutique comprenant le cis-oxalate (1R,2R-diaminocyclohexane) platine (II).

9. Composition pharmaceutique selon la revendication 7 ou 8, dans laquelle le cis-oxalate (1R, 2R-diaminocyclohexane) platine (II), le tégafur, le giméracil et l'otéracil de potassium sont présents dans un rapport molaire de (0,1-5) : 1 : (0,1-5) : (0,1-5).

10. Composition pharmaceutique selon la revendication 9, dans laquelle le rapport molaire est de (0,1 - 5) : 1 : 0,4 : 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2557303 B
- JP 2614164 B
- JP 8169825 A
- JP 2002205945 A

- US 6602870 B
- JP 60041077 A
- JP 49010510 A

### Non-patent literature cited in the description

- *Journal of Clinical Oncology,* 2004, vol. 22, 22-30
- *JOURNAL OF CLINICAL ONCOLOGY,* 2004, vol. 21, 2059-2069
- *JOURNAL OF CLINICAL ONCOLOGY,* 2000, vol. 18, 2938-2947

- *Journal of Clinical Oncology,* 2004, vol. 22, 2084-2091
- **M. MALET-MARTINO et al.** *The Oncologist,* 2002, vol. 7, 288-323
- **W. SCHEITHAUER et al.** *Colorectal Disease,* 2003, vol. 5 (3), 36-44
- *Statistical Science,* 1996, vol. 11 (4), 283-319